# EUROPEAN PATENT SPECIFICATION

(11) **EP 3 773 760 B1**
(45) Date of publication and mention of the grant of the patent: **22.04.2026**
(21) Application number: 19715514.6
(22) Date of filing: 10.04.2019
(51) Int. Cl.: A61L 9/12, B60H 3/00, B32B 29/08, B32B 27/10, B32B 27/36, B32B 27/40, B32B 27/06, A61L 9/04, B32B 27/08, B32B 7/06

(54) **DEVICE FOR DIFFUSING VOLATILE SUBSTANCES AND METHOD FOR ITS MANUFACTURING**
VORRICHTUNG ZUR DIFFUSION VON FLÜCHTIGEN SUBSTANZEN UND VERFAHREN ZU DEREN HERSTELLUNG
DISPOSITIF DE DIFFUSION DE SUBSTANCES VOLATILES ET SON PROCÉDÉ DE FABRICATION

(30) Priority: 12.04.2018 EP 18166958
(43) Date of publication of application: 17.02.2021
(73) Proprietor: Zobele Holding SpA, 38123 Trento (IT)
(72) Inventor: DEFLORIAN, Stefano, 38123 TRENTO (IT); DALSER, Alessio, 38123 TRENTO (IT); MANTOVAN, Stefano, 38123 TRENTO (IT)
(74) Representative: Herrero & Asociados, S.L.
(86) International application number: PCT/EP2019/059069
(87) International publication number: WO 2019/197458

(56) References cited:
- WO-A1-94/27646
- US-A- 4 529 124
- US-A1- 2014 193 295

## Description

The present invention refers to a device for diffusing volatile substances, comprising a substrate impregnated with said volatile substances. The present invention also refers to a method for manufacturing a device for diffusing volatile substances.

### Background of the invention

Impregnated carton with volatile substance are known for years. The simplest but still most popular application of this kind of product are car air fresheners consisting of an impregnated carton with an aesthetical artwork, impregnated with a fragrance and that is hanging from the rear mirror of the car. The main advantage of this kind of product is the cost.

Another advantage of the simple cellulosic support is that it is quite easy to manufacture, as the cellulose is quite absorbent. The amount of fragrance to be impregnated is simply put on the cellulose in horizontal position and after some seconds, is completely absorbed by the cellulose and then packed in an impermeable plastic film.

The olfactive performance is taking advantage of the generosity of the cellulose to release fragrance, but also presents the limitation of any open system that is that the high volatility components of the fragrance are released extremely fast and the low volatility remains longer. This way the smell of the device is progressively decaying and changing in its hedonic notes.

Several intends have been done to try to improve the performance with a limited cost increase. An example is US8968647B2 where a permeable membrane is applied on the top of the substrate, in order to control (reduce) the evaporation. Nevertheless, these improved versions are not fully satisfactory. On one side, the use of a semipermeable membrane does not only delay the release, it also filters the components of the fragrance, leading thus in a different fragrance.

Also, the presence of a plastic layer (impermeable or permeable) covering partially or completely the cardboard limits the possibility to impregnate the substrate with the fragrance. Even if the cardboard is only partially covered by the film, the quantity of free cardboard where to deposit the liquid will be reduced that definitively leads to a reduction of the impregnation speed and thus of the productivity.

US4529124A discloses a device for dispensing a volatile substance into the environment that includes a reservoir layer, having opposing first and second surfaces, for holding the substance and first and second envelope layers respectively in close proximity to the first surface and the second surface of the reservoir layer.

WO9427646A1 discloses a device for delivery of volatile materials comprising a volatile material delivery layer, a support layer and a repositionable adhesive layer. Devices so constructed may be applied to any desired substrate without fear of harming the surface of the substrate.

US2014193295A1 discloses a multi-layer article is containing a sorbent layer having a first side and a second side; a vapor permeable or microporous layer adjacent to at least a portion of at least one of the first side and second side of the sorbent layer.

Therefore, one purpose of the present invention is to provide a device for diffusing volatile substances achieving a more continuous release and a method for manufacturing such device.

### Description of the invention

With the device for diffusing volatile substances according to the present invention it is possible to solve said drawbacks, providing other advantages that are described below.

The device for diffusing volatile substances according to the present invention is defined in claim 1, and it comprises a substrate with fibers impregnated with volatile substances, and the device comprises a first film that is impermeable to the volatile substances applied to one face of the substrate and a second film provided with a plurality of holes applied to the other face of the substrate, wherein the second film is heat sealed on the substrate, so that the second film is fixed to the fibers of the substrate.

Preferably, said second film is impermeable to said volatile substances, even though it could also be semipermeable to said volatile substances.

The diameter of the holes is between 0.5 cm and 2 cm, and the separation between two adjacent holes is less than the double of the diameter of the holes.

Preferably, the first film and the second film have a thickness between 20 and 100 microns, the substrate is made from cardboard, and the substrate has a thickness between 1 and 2 mm.

According to a second aspect, the invention also refers to a method for manufacturing a device for diffusing volatile substances that is defined in claim 6, said device comprising a substrate with fibers, the method comprising the following steps:
- applying a first film to a face of the substrate;
- impregnating the substrate by depositing the volatile substances on the face of the substrate that is not coated with the first film;
- leaving the volatile substances to fully impregnate the substrate; and
- applying a second film provided with a plurality of holes to the other face of the substrate.

The method also comprises, after the application of the second film, the step of sealing the second film by heat sealing, so that the second film is fixed to the substrate, and the diameter of the holes is between 0.5 cm and 2 cm, and the separation between two adjacent holes is less than the double of the diameter of the holes.

For preventing the undesired diffusion of the volatile substances during the manufacturing of the device, the method also comprises, during the application of the second film, using a liner covering the second film.

The liner can be a film that is placed between the top film and the welding tool during welding, and then is only in contact with the product during the welding step.

Alternatively, the liner can be a continuous film on which the top film is placed before to enter in the welding station
Still alternatively, the liner and the top film are laminated together off line, as two continuous layers. The holes in the top film are made by laser cutting, remaining the plastic film of the inner of the holes on the liner, welding the multilayer liner/top film without applying pressure and heat on the areas corresponding to the inner of the holes, then removing the liner, leaving the top film on top of the substrate but removing the inner of the holes together with the liner. This last operation of separating liner from the top film can be done during the production process or by the consumer.

The device for diffusing volatile substances according to the present invention permits a more continuous diffusion of the volatile substances along time.

### Brief description of the drawings

For a better understanding the above explanation and for the sole purpose of providing an example, some non-limiting drawings are included that schematically depict a practical embodiment.

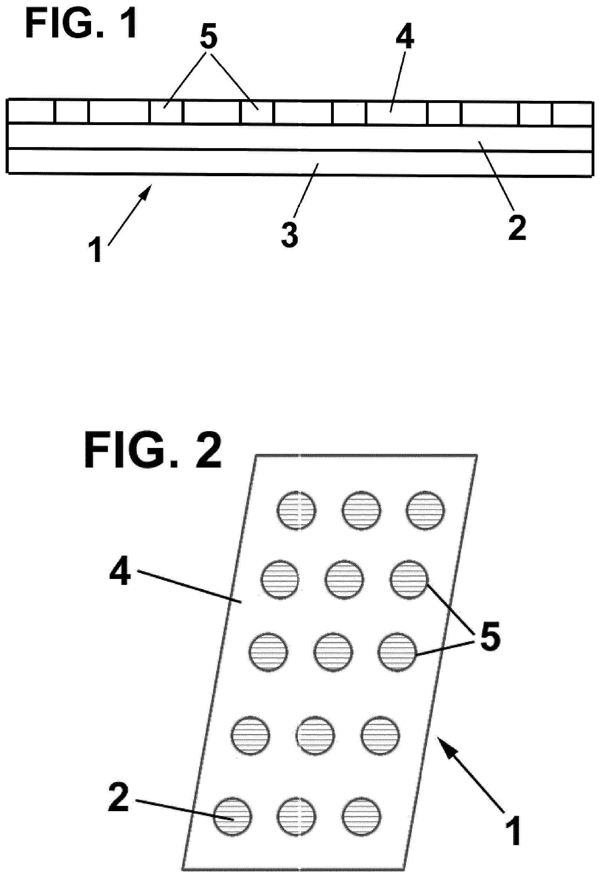

Figure 1 is a cross-section view of the device for diffusing volatiles substances according to the present invention; and
Figure 2 is a frontal view of the device for diffusing volatiles substances according to the present invention.

### Description of a preferred embodiment

The present invention refers to a device for diffusing volatile substances, indicated generally by numeral reference 1, comprising a substrate 2 impregnated with volatile substances, covered on one side by a first film 3 and on the other side by a second film 4 in which a plurality of holes 5 has been done.

According to a preferred embodiment, the substrate 2 is a cardboard substrate, the first film 3 is an impermeable plastic film and the second film 4 is preferably also impermeable to fragrances, although a permeable film could also be used, assuming this will lead to a lower performance results, as the volatile substances will be filtered in the area where it is covered by this permeable film.

The evaporation will take place mainly from the holes 5 done in the second film 4, as we consider the evaporation of the outer perimeter of the substrate 2 (that is not covered by any film) as very low and limited to the first hours of the product.

The part of the substrate 2 that is not covered by the first or second films 3, 4 will behave like a typical cardboard substrate 2 without any film and then release the volatile substance without any limit, and so with the decompensation of fragrance components as described above.

The part of the substrate 2 that is covered by the first or second films 3, 4 will not contribute directly to the evaporation, but will feed progressively the not covered areas with fresh doses of the volatile substance that will evaporate from there.

The resulting evaporation will have an initial lower evaporation rate in comparison with a standard impregnated substrate with the same dimensions but followed by a lower decay in weight loss per hour.

If we consider a fragrance, the initial lower evaporation rate can be compensated by using a fragrance with a higher volatility.

The size of the holes 5 in the second film 4 is in the range from 0.5 to 2 cm, and more preferably around 1 cm.

The distance between two holes remains below the double of the size of the holes 5 and also below 3 cm.

The thickness of the substrate 2 is preferably from 1 to 2 mm, more preferably, 1.5 mm.

The thickness of the first and second films 3, 4, that are preferably of the same material, is between 20 to 100 microns. The first and second films 3, 4 are preferably multilayer films, with a barrier material, for example PET, maybe complemented by an EVOGH layer, and a heat sealing layer, preferably made of polyethylene, polypropylene, or ionomer resin.

The device for diffusing volatile substances 1 according to the present invention is packed in an impermeable envelope (not shown in the drawings) that avoid any fragrance loss before a first use.

The present invention also refers to a method for manufacturing a device for diffusing volatile substances as described previously.

According to a preferred embodiment, this method comprises the following steps:
- applying the first film 3 to a face of the substrate 2;
- impregnating the substrate 2 by depositing the volatile substances on the side of the substrate 2 that is not coated with the first film 3;
- leaving the volatile substances to fully impregnate the substrate 2;
- applying the second film 4 to the other face of the substrate 2;
- sealing the second film 4 by heat sealing, so that the second film 4 is fixed to the fibers of the substrate 2.

The heat necessary to seal the second film 4 will influence the volatile substances. On one side, it can provoke unwanted evaporation of part of the volatile substances; on the other side, it can degrade part of the volatile substances.

In the case of an air freshener, even a small portion of the substance degraded can provoke a significant change in the olfactive note.

For this reason, it is preferred to apply the second film 4 with a liner that covers the whole second film 4, in order the volatile substances are never directly exposed to the heating plate of the sealing tool, and there is no exit for the volatile substances to evaporate from the second film 4 before the liner is removed.

The liner consists in a plastic film that is not sensitive to the temperature used during the sealing of the second film 4 on the substrate 2. Preferably, the liner is made of a polyester film with a layer between the liner and the second film 4 made of polyurethane resin.

Even though reference has been made to a specific embodiment of the invention, it is obvious for a person skilled in the art that the device described herein is susceptible to numerous variations and modifications, and that all the details mentioned can be substituted for other technically equivalent ones without departing from the scope of protection defined by the attached claims.

## Claims

1. Device (1) for diffusing volatile substances comprising a substrate (2) with fibers impregnated with volatile substances, the device comprising a first film (3) that is impermeable to the volatile substances applied to one face of the substrate (2) and a second film (4) provided with a plurality of holes (5) applied to the other face of the substrate (2),
wherein the second film (4) is heat sealed, so that the second film (4) is fixed to the fibers of the substrate (2),
**characterized in that** the diameter of the holes (5) is between 0.5 cm and 2 cm, and the separation between two adjacent holes (5) is less than the double of the diameter of the holes (5).

2. Device (1) for diffusing volatile substances according to claim 1, wherein said second film (4) is impermeable to said volatile substances.

3. Device (1) for diffusing volatile substances according to claim 1, wherein the first film (3) and the second film (4) have a thickness between 20 and 100 microns.

4. Device (1) for diffusing volatile substances according to claim 1, wherein the substrate (2) is made from cardboard.

5. Device (1) for diffusing volatile substances according to claim 1 or 4, wherein the substrate (2) has a thickness between 1 and 2 mm.

6. Method for manufacturing a device (1) for diffusing volatile substances according to any of the previous claims, said device (1) comprising a substrate (2) with fibers, the method comprising the following steps:
- applying a first film (3) to a face of the substrate (2);
- impregnating the substrate (2) by depositing the volatile substances on the face of the substrate (2) that is not coated with the first film (3);
- leaving the volatile substances to fully impregnate the substrate (2); and
- applying a second film (4) provided with a plurality of holes (5) to the other face of the substrate (2),
wherein the method also comprises, after the application of the second film (4), the step of sealing the second film (4) by heat sealing, so that the second film (4) is fixed to the fibers of the substrate (2),
**characterized in that** the diameter of the holes (5) is between 0.5 cm and 2 cm, and the separation between two adjacent holes (5) is less than the double of the diameter of the holes (5).

7. Method for manufacturing a device (1) for diffusing volatile substances according to claim 6, wherein the method also comprises, during the application of the second film (4), using a liner covering the second film (4).

## Patentansprüche

1. Vorrichtung (1) zum Diffundieren flüchtiger Substanzen, umfassend ein Substrat (2) mit Fasern, die mit flüchtigen Substanzen imprägniert sind, wobei die Vorrichtung einen ersten Film (3), der für die flüchtigen Substanzen undurchlässig ist und auf einer Seite des Substrats (2) angebracht ist, und einen zweiten Film (4) mit einer Vielzahl von Löchern (5) umfasst, der auf der anderen Seite des Substrats (2) angebracht ist,
wobei der zweite Film (4) heißversiegelt ist, so dass der zweite Film (4) an den Fasern des Substrats (2) befestigt ist,
**dadurch gekennzeichnet, dass** der Durchmesser der Löcher (5) zwischen 0,5 cm und 2 cm liegt und der Abstand zwischen zwei benachbarten Löchern (5) weniger als das Doppelte des Durchmessers der Löcher (5) beträgt.

2. Vorrichtung (1) zum Diffundieren flüchtiger Substanzen gemäß Anspruch 1, wobei der zweite Film (4) für die flüchtigen Substanzen undurchlässig ist.

3. Vorrichtung (1) zum Diffundieren flüchtiger Substanzen gemäß Anspruch 1, wobei der erste Film (3) und der zweite Film (4) eine Dicke zwischen 20 und 100 Mikrometern aufweisen.

4. Vorrichtung (1) zum Diffundieren flüchtiger Substanzen gemäß Anspruch 1, wobei das Substrat (2) aus Pappe gemacht ist.

5. Vorrichtung (1) zum Diffundieren flüchtiger Substanzen gemäß Anspruch 1 oder 4, wobei das Substrat (2) eine Dicke zwischen 1 und 2 mm aufweist.

6. Verfahren zur Herstellung einer Vorrichtung (1) zum Diffundieren flüchtiger Substanzen gemäß einem der vorstehenden Ansprüche, wobei die Vorrichtung (1) ein Substrat (2) mit Fasern umfasst, wobei das Verfahren die folgenden Schritte umfasst:
- Aufbringen eines ersten Films (3) auf eine Seite des Substrats (2);
- Imprägnieren des Substrats (2) durch Aufbringen der flüchtigen Substanzen auf die Seite des Substrats (2), die nicht mit dem ersten Film (3) beschichtet ist;
- Belassen der flüchtigen Substanzen, damit sie das Substrat (2) vollständig imprägnieren; und
- Aufbringen eines zweiten Films (4) mit einer Vielzahl von Löchern (5) auf die andere Seite des Substrats (2),
wobei das Verfahren nach dem Aufbringen des zweiten Films (4) auch den Schritt des Versiegelns des zweiten Films (4) durch Heißsiegeln umfasst, so dass der zweite Film (4) an den Fasern des Substrats (2) befestigt wird,
**dadurch gekennzeichnet, dass** der Durchmesser der Löcher (5) zwischen 0,5 cm und 2 cm liegt und der Abstand zwischen zwei benachbarten Löchern (5) weniger als das Doppelte des Durchmessers der Löcher (5) beträgt.

7. Verfahren zur Herstellung einer Vorrichtung (1) zum Diffundieren flüchtiger Substanzen gemäß Anspruch 6, wobei das Verfahren während des Aufbringens des zweiten Films (4) auch die Verwendung einer den zweiten Film (4) bedeckenden Auskleidung umfasst.

## Revendications

1. Dispositif (1) de diffusion de substances volatiles comprenant un substrat (2) à fibres imprégnées de substances volatiles, le dispositif comprenant un premier film (3) imperméable aux substances volatiles appliqué sur une face du substrat (2) et un second film (4) doté d'une pluralité de trous (5) appliqués sur l'autre face du substrat (2),
dans lequel le second film (4) est thermoscellé, de sorte que le second film (4) est fixé aux fibres du substrat (2),
**caractérisé en ce que** le diamètre des trous (5) est compris entre 0,5 cm et 2 cm, et la séparation entre deux trous (5) adjacents est inférieure au double du diamètre des trous (5).

2. Dispositif (1) de diffusion de substances volatiles selon la revendication 1, dans lequel ledit second film (4) est imperméable auxdites substances volatiles.

3. Dispositif (1) de diffusion de substances volatiles selon la revendication 1, dans lequel le premier film (3) et le second film (4) ont une épaisseur comprise entre 20 et 100 microns.

4. Dispositif (1) de diffusion de substances volatiles selon la revendication 1, dans lequel le substrat (2) est en carton.

5. Dispositif (1) de diffusion de substances volatiles selon la revendication 1 ou 4, dans lequel le substrat (2) présente une épaisseur comprise entre 1 et 2 mm.

6. Procédé de fabrication d'un dispositif (1) de diffusion de substances volatiles selon l'une quelconque des revendications précédentes, ledit dispositif (1) comprenant un substrat (2) à fibres, le procédé comprenant les étapes suivantes :
- l'application d'un premier film (3) sur une face du substrat (2) ;
- l'imprégnation du substrat (2) par dépôt des substances volatiles sur la face du substrat (2) qui n'est pas revêtue du premier film (3) ;
- le fait de laisser les substances volatiles imprégner complètement le substrat (2) ; et
- l'application d'un second film (4) doté d'une pluralité de trous (5) sur l'autre face du substrat (2),
dans lequel le procédé comprend également, après l'application du second film (4), l'étape de scellement du second film (4) par thermoscellement, de sorte que le second film (4) soit fixé aux fibres du substrat (2),
**caractérisé en ce que** le diamètre des trous (5) est compris entre 0,5 cm et 2 cm, et la séparation entre deux trous (5) adjacents est inférieure au double du diamètre des trous (5).

7. Procédé de fabrication d'un dispositif (1) de diffusion de substances volatiles selon la revendication 6, dans lequel le procédé comprend également, lors de l'application du second film (4), l'utilisation d'un revêtement couvrant le second film (4).
